(19) Europäisches Patentamt
European Patent Office
Office européen des brevets

(11) EP 0 919 244 A2

(12) **EUROPÄISCHE PATENTANMELDUNG**

(43) Veröffentlichungstag:
**02.06.1999 Patentblatt 1999/22**

(51) Int. Cl.$^6$: **A61K 45/06**

(21) Anmeldenummer: **98250357.5**

(22) Anmeldetag: **09.10.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **10.10.1997 DE 19744676**

(71) Anmelder:
**Forschungszentrum Borstel Zentrum für Medizin und Biowissenschaften**
**23845 Borstel (DE)**

(72) Erfinder:
- **Van der Bosch, Jürgen, Dr.**
**23552 Lübeck (DE)**
- **Schlaak, Max, Prof. Dr.**
**24119 Kronshagen (DE)**
- **Rüller, Stephan, Dr.**
**22941 Bargteheide (DE)**

(74) Vertreter: **UEXKÜLL & STOLBERG**
**Patentanwälte**
**Beselerstrasse 4**
**22607 Hamburg (DE)**

# (54) Kombinationspräparate für die Therapie von Tumoren

(57) Die Erfindung betrifft Erzeugnisse, die einen Hemmer cyclinabhängiger Proteinkinasen und einen oder mehrere Modulatoren mindestens eines weiteren proteinmodifizierenden Enzyms als Kombinationspräparat zur gleichzeitigen, getrennten oder zeitlich abgestuften Anwendung als Arzneimittel enthalten. Die Erzeugnisse eignen sich insbesondere zu Anwendung in der Tumortherapie.

Abb. 1:
Einzelsubstanzen

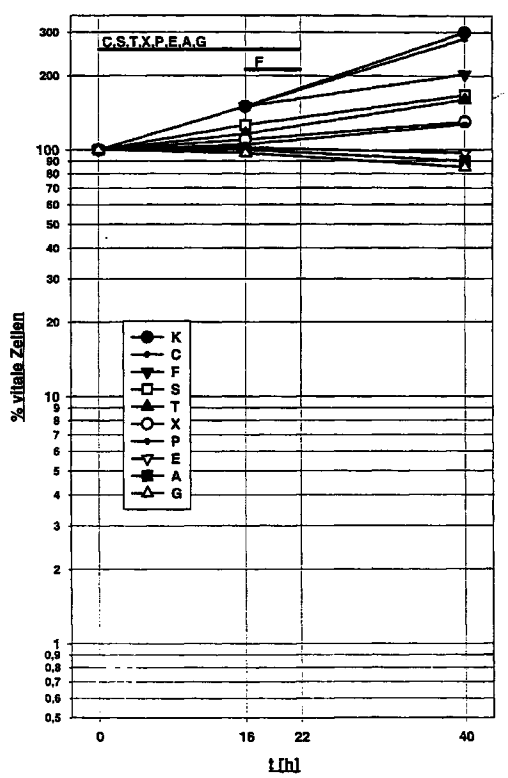


EP 0 919 244 A2

## Beschreibung

Die vorliegende Erfindung betrifft Arzneimittel zur Behandlung von Tumoren, die eine synergistisch wirkende Kombination aus einem Hemmer cyclinabhängiger Proteinkinasen und einem Modulator mindestens eines weiteren proteinmodifizierenden Enzyms enthalten.

Zentrales Ziel der Tumortherapie ist die selektive Eliminierung neoplastisch transformierter Zellen. Bei der klassischen Chemotherapie werden hierzu Wirkstoffe eingesetzt, die direkt mit den Mechanismen der DNA-Replikation oder der Mitose wechselwirken, beispielsweise durch Hemmen der Nukleotidbiosynthese, Verändern der DNA-Struktur durch Interkalation, Alkylierung oder Blockieren der an der Replikation beteiligten Enzyme, wie beispielsweise der Topoisomerasen, oder dadurch, daß sie als Mitose-Spindelgifte wirken. Da es sich bei diesen Prozessen um Vorgänge handelt, die auch für normale Zellen überlebenswichtig sind, ist die herkömmliche Chemotherapie meist mit schweren Nebenwirkungen verbunden.

Darüber hinaus ist die Verwendung von Zytokinen zur Induktion des Zelltods von Tumorzellen bekannt. Zytokine greifen unter anderem in die für die zelluläre Proliferation verantwortliche intrazelluläre Signaltransduktion ein. Der Zytokin-induzierte Zelltod wird von einer Fragmentierung der genomischen DNA begleitet, die zu einem irreversiblen Verlust genetischer Information führt und somit vermutlich Ursache für die Irreversibilität des Todesereignisses ist.

Obwohl durch die Verwendung von Kombinationen verschiedener Zytokine *in vitro* der Zelltod unterschiedlicher Tumorzellinien ausgelöst werden konnte, sind Zytokine aufgrund hoher Nebenwirkungen wie Schock oder Multiorganversagen in der Regel therapeutisch nicht anwendbar (R. A. Heller, M. Krönke, The Journal of Cell Biology **126** (1994) 5; R. Beyaert, W. Fiers, FEBS Letters **340** (1994) 9).

Die Wirkung der Zytokine läßt sich durch gleichzeitige Gabe von Hemmern der Proteinbiosynthese, wie beispielsweise Cycloheximid, verstärken (R. A. Heller, M. Krönke, aaO; R. Beyaert, W. Fiers, aaO).

M. Weil et al., J. Cell. Biol. 133 (1996) 1053, beschreiben die Auslösung des programmierten Zelltodes durch Verwendung von Cycloheximid und dem Proteinkinasehemmer Staurosporin. Durch Anwendung hoher Staurosporinkonzentrationen konnte in Anwesenheit von Cycloheximid der Tod unter anderem von normalen Nieren- und Lungenzellen induziert werden. Arzneimittel oder Mittel, welche die selektive Bekämpfung von Tumorzellen erlauben, werden nicht beschrieben.

Die EP 0 366 061 B1 offenbart die Verwendung von 4H-1-Benzopyran-4-on-Derivaten zur Bekämpfung von Tumorerkrankungen.

Die EP 0 464 656 A1, EP 0 612 728 A2 und J62089659 A offenbaren neue Anisomycin-Derivate, die sich unter anderem zur Behandlung von Tumoren eignen.

Die EP A 0 196 415 und die J60149520 A offenbaren die Verwendung von Trichostatin A und C als Antitumorwirkstoffe.

J` Taunton et al., J. Am. Chem. Soc. 118 (1996) 10412 beschreiben die Synthese natürlicher oder modifizierter Trapoxin-Derivate, welche als Inhibitoren der Histondeacetylase wirken.

K.C. Bible und S.H. Kaufmann, Cancer Research 56 (1996) 4856 haben nachgewiesen, daß Flavopiridol zytotoxisch auf Lungenkarzinomzellen wirkt, die Zytotoxizität jedoch durch gleichzeitige Gabe von Cycloheximid herabgesetzt wird.

Aufgabe der Erfindung ist die Schaffung von Arzneimitteln zur Behandlung von Tumorerkrankungen, die eine im Vergleich zu bekannten Mitteln erhöhte Wirksamkeit aufweisen und nicht die mit der herkömmlichen Chemotherapie verbundenen Nebenwirkungen zeigen.

Diese Aufgabe wird überraschend durch Erzeugnisse gelöst, die einen Hemmer cyclinabhängiger Proteinkinasen und einen oder mehrere Modulatoren mindestens eines weiteren proteinmodifizierenden Enzyms als Kombinationspräparat zur gleichzeitigen, getrennten oder zeitlich abgestuften Anwendung enthalten.

Hemmer cyclinabhängiger Proteinkinasen im Sinne der Erfindung sind Substanzen, deren $IC_{50}$ bezüglich der Aktivität einer cyclinabhängigen Kinase $\leq 10\ \mu M$, vorzugsweise $\leq 1\ \mu M$ ist.

Bevorzugte Hemmer cyclinabhängiger Proteinkinasen sind die in der EP 0 366 061 A1 offenbarten 4H-1-Benzopyran-4-on-Derivate sowie deren pharmakologisch verträgliche Säureadditionssalze. Hierbei handelt es sich um Substanzen gemäß der Formel

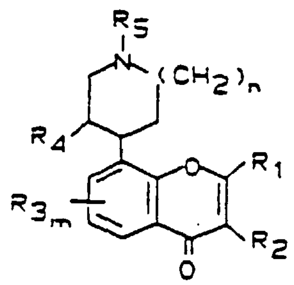

in der

- $R^1$ Wasserstoff, $C_1$-$C_6$-Alkyl, Aryl-$C_1$-$C_4$-alkyl, substituiertes $C_1$-$C_6$-Alkyl, $C_3$-$_6$-Cycloalkyl, einen $C_3$-$C_9$-Heterozyklus mit 1, 2 oder 3 Heteroatomen wie N, S, O oder einer beliebigen Kombinationen dieser Atome, $C_3$-$C_6$-Cycloalkyl-$C_1$-$C_4$-alkyl, $C_2$-$C_6$-Alkenyl, $C_2$-$C_6$-Alkinyl, Aryl (einschließlich polycyclischer Ringe), einen aromatischen heterocyclischen Rest, substituiertes Aryl, -COOH, eine Aldehyd-$C_1$-$C_4$-alkylgruppe, eine COO-$C_1$-$C_4$-alkylgruppe, eine primäre Amino-, Alkylamino-, Aralkylamino-, Dialkylamino-, Amido-, Arylamino-, Diarylaminogruppe oder -$CH_2$O-$C_1$-$C_4$-Alkyl bedeutet;
- $R^2$ Wasserstoff oder $C_1$-$C_6$-Alkyl, Aryl, Nitro, Amino, Di-$C_1$-$C_4$-alkylamino, Halogen, Hydroxy, Alkoxy, -COOH, -COO-$C_1$-$C_4$-Alkyl, -CHO, -$CH_2$OH oder -$CH_2$O-$C_1$-$C_4$-Alkyl bedeutet;
- $R^3$ Wasserstoff oder $C_1$-$C_4$-Alkyl, substituiertes $C_1$-$C_4$-Alkyl, Hydroxy, -COOH, Nitro, eine Amino-, $C_1$-$C_4$-Alkylamino-, Di-$C_1$-$C_4$-alkylaminogruppe, Halogen, O-Alkyl-C(=0)-alkyl, -CHO, -$CH_2$OH, -$CH_2$O-$C_1$-$C_4$-Alkyl, $R_2$N-C(=0)-O- bedeutet; wobei
- R H, $C_1$-$C_6$-Alkyl, Cycloalkyl O-Alkyl-C(=O)-alkyl oder Aryl ist;
- $R^4$ Wasserstoff, Hydroxy, $C_1$-$C_4$-Alkoxy, $C_1$-$C_4$-Alkanoyloxy, $C_1$-$C_4$-Alkoxycarbonyl, Aryloxy, eine Amino-, $C_1$-$C_4$-Alkylamino-, Di-$C_1$-$C_4$-alkylaminogruppe, $R_2$N-C(=O)-O-, bedeutet und R die oben angegebene Bedeutung hat;
- $R^5$ Wasserstoff, $C_1$-$C_6$-Alkyl, Aryl-$C_1$-$C_4$-alkyl, $C_3$-$C_6$-Cycloalkyl, $C_3$-$C_6$-Cycloalkyl-$C_1$-$C_4$-alkyl, Alkylamino-, $C_1$-$C_4$-Alkanoyl, -C(=O)-O-$C_1$-$C_4$-Alkyl oder Aroyl bedeutet, wobei die Arylgruppe ein unsubstituierter oder mono-oder polysubstituierter Phenylrest ist.
- m eine ganze Zahl zwischen 0 und 3; und
- n eine ganze Zahl zwischen 0 und 2 ist.

Unabhängig voneinander wählbare bevorzugte Definitionen sind

- $R^1$ H, $C_1$-$C_3$-Alkyl, Naphthyl, Aryl, subsituiertes Aryl, Aralkyl oder ein aliphatischer oder aromatischer $C_3$-$C_9$-Heterocyclus mit 1, 2 oder 3 Heteroatomen wie N, S, O oder einer beliebigen Kombination dieser Atome;
- $R^2$ H oder $C_1$-$C_3$-Alkyl;
- $R^3$ OH oder $OCH_3$;
- $R^4$ OH;
- $R^5$ $C_1$-$C_3$-Alkyl, $C_3$-$C_5$-Cycloalkyl, $C_3$-$C_5$-Cycloalkyl-$C_1$-$C_4$-Alkyl;

m = 1 oder 2; und/oder
n = 1 ist.

Besonders bevorzugt sind Verbindungen der Formel

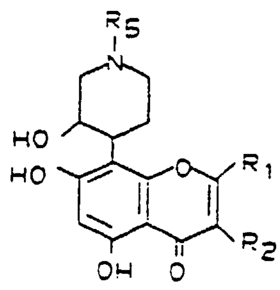

in der

$R^1$ Wasserstoff, $C_1$-$C_3$-Alkyl, Naphthyl, Aryl, Aralkyl, substituiertes Aryl, einen $C_3$-$C_9$-Heterocyclus oder einen aromatischen Heterocyclus bedeutet;
$R^2$ Wasserstoff oder $C_1$-$C_3$-Alkyl bedeutet;
$R^5$ $C_1$-$C_3$-Alkyl, $C_3$-$C_5$-Cycloalkyl, $C_3$-$C_5$-Cycloalkyl-$C_1$-$C_4$-Alkyl bedeutet.

Ganz besonders bevorzugt sind Verbindungen bei denen

$R^1$ Phenyl, Thienyl, Pyridyl, Chlorphenyl, Dichlorphenyl, Methylphenyl, Aminophenyl, Bromphenyl, Hydroxyphenyl oder Naphthyl bedeutet;
$R^2$ Wasserstoff bedeutet; und
$R^5$ Methyl bedeutet.

"Alkyl" steht vorzugsweise für geradkettige oder verzweigte Radikale mit 1 bis 6, besonders bevorzugt 1 bis 5 Kohlenstoffatomen, insbesondere Methyl-, Ethyl-, Propyl-, Isopropyl-, t-Butyl-, Pentyl- oder Isopentylgruppen.

"Substituiertes Alkyl" steht vorzugsweise für Halogenalkyl, wie Trifluormethylalkyl, Hydroxyalkyl, wie Hydroxyethyl, oder Carboxyalkyl, wie Carboxyethyl.

"Cycloalkyl" steht vorzugsweise für Gruppen mit 3 bis 6 Kohlenstoffatomen, insbesondere Cyclopropyl, Cyclobutyl, Cyclopentyl oder Cyclohexyl. Eine bevorzugte Cycloalkylalkylgruppe ist Cyclopropylmethyl.

Bevorzugte Aralkylgruppen sind unsubstituierte oder durch Substituenten wie Halogen, $C_1$-$C_4$-Alkyl, $C_1$-$C_4$-Alkoxy, Nitro- oder Trifluormethylgruppen, Aminogruppen und/oder substituierte Aminogruppen einfach oder mehrfach substituierte Phenylgruppen.

"Aryl" steht für unsubstituierte und einfach- oder mehrfach substituierte Phenylgruppen. Bevorzugte Substituenten sind Halogen, $C_1$-$C_4$-Alkyl, $C_1$-$C_4$-Alkoxy, Hydroxy, Carboxy, COO-Alkyl, $CONH_2$, CONH-Alkyl, $CON(Alkyl)_2$, Nitro, Trifluormethyl, Amino, $C_1$-$C_4$-Alkylamino, Di-$C_1$-$C_4$-alkylamino, aromatische Heterocyclen wie Pyridylgruppen oder polycyclische aromatische Reste wie Naphthylgruppen.

Bevorzugte Alkylaminogruppen sind $(CH_2)_p$-$NR^6R^7$, wobei p = 1 bis 3 ist. $R^6$ und $R^7$ sind Alkylgruppen, wobei Alkyl die oben angegebene Bedeutung hat, oder bilden zusammen mit dem Stickstoffatom, an das sie gebunden sind, einen heterocyclischen Ring mit einem oder mehreren Heteroatomen. Bevorzugte heterocyclische Ringe sind Piperidin, Pyrrolidin, Morpholin, Piperazin und Imidazol. Die heterocylischen Ringe können unsubstituiert oder ein- oder mehrfach durch $C_1$-$C_4$-Alkyl, $C_1$-$C_4$-Alkoxy, Aryl, Hydroxyl- oder Aminogruppe substituiert sein.

Geeignete Beispiele für Salze der erfindungsgemäßen Verbindungen mit anorganischen oder organischen Säuren sind das Hydrochlorid, Hydrobromid, Sulfat, Phosphat, Acetat, Oxalat, Tartrat, Citrat, Maleat oder Fumarat.

Der am meisten bevorzugte Hemmer cyclinabhängiger Proteinkinasen ist Flavopiridol ((-)cis-5,7-Dihydroxy-2-(2-chlorphenyl)-8-(4-(3-hydroxy-1-methyl)-piperidinyl)-4H-1-benzopyran-4-on)).

Proteinmodifizierende Enzyme im Sinne der Erfindung sind beispielsweise streßaktivierte Proteinkinasen, Histondeacetylase, die an der Proteinbiosynthese beteiligten Enzyme sowie Proteinphosphatasen und Proteinkinasen.

Bevorzugte Modulatoren proteinmodifizierender Enzyme sind Aktivatoren streßaktivierter Proteinkinasen, Hemmer der Histondeacetylase, Hemmer der Proteinbiosynthese und Hemmer von Proteinphosphatasen sowie weiterer, d.h. nicht streßaktivierter oder cyclinabhängiger, Proteinkinasen, wie beispielsweise Hemmer von Tyrosinkinasen und Breitband-Proteinkinase-Inhibitoren.

Als Aktivatoren streßaktivierter Proteinkinasen werden im Rahmen der Erfindung solche Substanzen bezeichnet, die direkt oder indirekt eine Aktivierung von streßaktivierten Proteinkinasen bewirken. Bevorzugt sind Aktivatoren, die in einer Konzentration von $\leq 1$ µM eine Aktivierung von $\geq 50$ % bewirken. Bei streßaktivierten Proteinkinasen

(SAPKs) handelt es sich um eine Gruppe von Proteinkinasen, die durch Streßreize wie beispielsweise UV-Bestrahlung aktiviert werden (J.R. Woodgett, J. Avruch, J. Kyriakis, Cancer Surveys **27** (1996) 127; S. Cosulich und P. Clarke, Current Biology **6** (1996) 1586; P. Cohen, Trends in Cell Biol. 7 (1997) 353).

Bevorzugte Aktivatoren streßaktivierter Proteinkinasen sind Anisomycin und die in der EP 0 464 656 A1 und EP 0 612 728 A2 offenbarten Anisomycin-Derivate sowie deren pharmakologisch verträgliche Salze. Hierbei handelt es sich um Substanzen gemäß der Formel

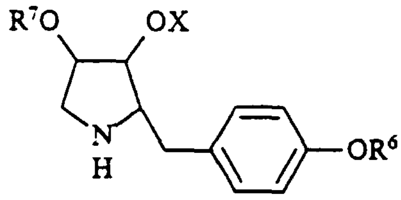

in der

| | |
|---|---|
| $R^6$ | Wasserstoff oder $C_1$-$C_6$-Alkyl bedeutet; |
| $R^7$ | Wasserstoff oder eine gesättigte oder ungesättigte, lineare, verzweigte oder cyclische $C_1$-$C_{18}$-Acylgruppe bedeutet; und |
| X | $C_1$-$C_6$-Alkyl-CO-; -CO-$NR^8R^9$ oder -$CH_2R^{10}$ bedeutet, wobei |
| $R^8$ und $R^9$ | unabhängig voneinander Wasserstoff, eine lineare oder cyclische $C_1$-$C_6$-Alkylgruppe, Phenyl, eine substituierte lineare oder verzweigte $C_1$-$C_6$-Alkylgruppe oder einen substituierten Phenylrest bedeuten; und |
| $R^{10}$ | Wasserstoff, $C_1$-$C_6$-Alkyl, $C_1$-$C_6$-Alkoxy oder eine substituierte $C_1$-$C_6$-Alkoxygruppe bedeutet. |

Bevorzugt sind solche Derivate, bei denen $R^6$ $CH_3$ bedeutet; $R^7$ $C_{1-12}$-Acyl bedeutet; X -C(=O)$NH_2$ oder -C(=O)$C_{1-6}$-Alkyl bedeutet; $R^8$ und $R^9$ H bedeuten; und $R^{10}$ H oder $C_{1-3}$-Alkyl bedeutet. Der am meisten bevorzugte Aktivator für streßaktivierte Proteinkinasen ist Anisomycin (2-p-Methoxyphenylmethyl-3-acetoxy-4-hydroxypyrrolidin).

Bevorzugte Hemmer der Histondeacetylasen sind pharmazeutisch verträgliche Salze der Buttersäure, Trichostatin A, Trichostatin C, Trapoxin, Chlamydocin, HC-Toxin, WP-3161 und/oder Cyt 2. Geeignete Verbindungen dieses Typs werden beispielsweise von J. Taunton et al., J. Am. Chem. Soc. **118** (1996) 10412 beschrieben.

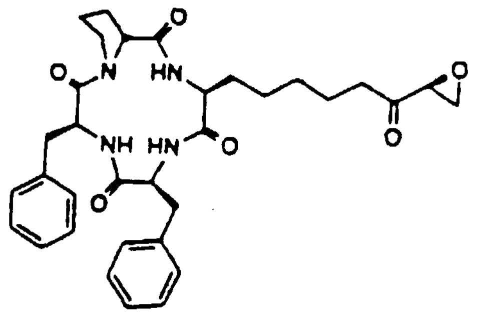

Trapoxin B

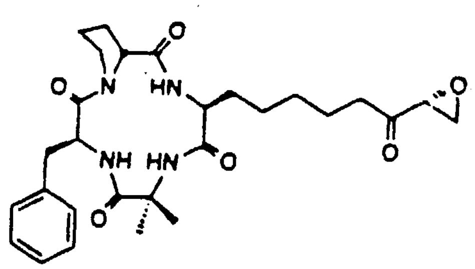

Chlamydoxin

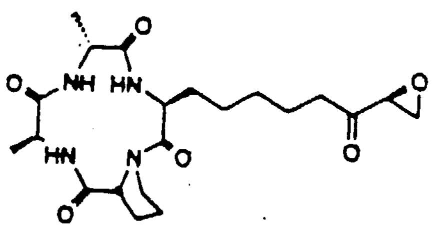

HC-Toxin

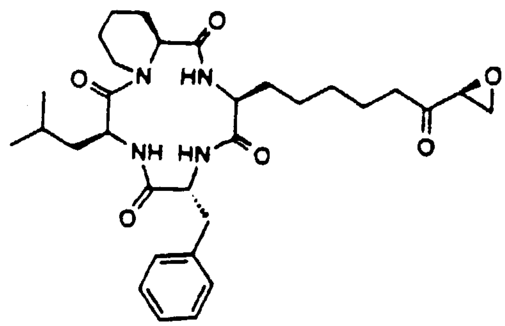

WP 3161

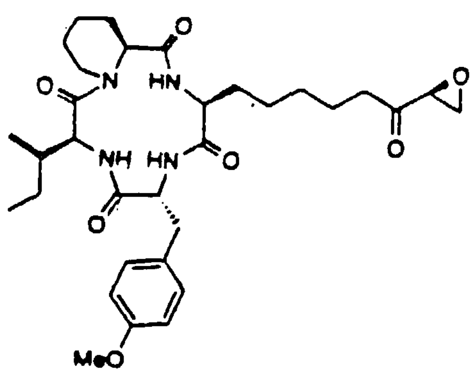

Cyt 2

Als Hemmer der Proteinbiosynthese sind Puromycin, Cycloheximid und/oder Emetin bevorzugt. Puromycin hemmt neben der Proteinbiosynthese zusätzlich eine Zellzyklus-regulatorische Protease (PSA = Puromycin-Sensitive Aminopeptidase).

Bevorzugte Hemmer von Proteinphosphatasen bzw. von Proteinkinasen sind Staurosporin, Staurosporin-Derivate, Cyclosporin A, Geldanamycin (GDA; R = $CH_3O$; -$C^4$=$C^5$-), Dihydrogeldanamycin (DHGDA; R = $CH_3O$; -$C^4H$-$C^5H$-) und/oder Geldanamycin- und Dihydrogeldanamycin-Derivate gemäß der Formel

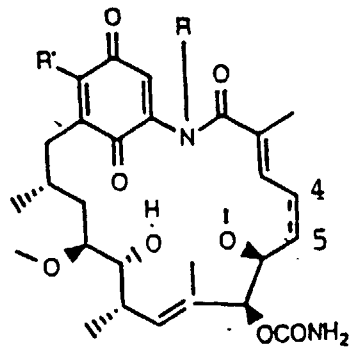

in der

R H ist und

R' eine primäre, sekundäre oder tertiäre Aminogruppe, wie eine einfach oder zweifach durch $C_{1\text{-}6}$-, vorzugsweise $C_{1\text{-}3}$-Alkyl- oder $C_{1\text{-}6}$-, vorzugsweise $C_{1\text{-}3}$-Alkenylreste substituierte Aminogruppe, insbesondere $-NH_2$, $-NH-(CH_2)_n-CH_3$,

$$-N<\begin{matrix}(CH_2)_m-CH_3\\ (CH_2)_n-CH_3\end{matrix}\quad,\quad -N<\begin{matrix}CH_2\\ |\\ (CH_2)_p\end{matrix}\quad \text{oder}\quad -NH-CH<\begin{matrix}CH_2\\ |\\ (CH_2)_p\end{matrix}$$

bedeutet, wobei n und m unabhängig voneinander 0 bis 6 und p 1 bis 7, vorzugsweise 1 bis 4 sind.

Geeignete GDA- und DHGDA-Derivate werden beispielsweise von R.C. Schnur et al., J. Med. Chem. **38** (1995) 3806; R.C. Schnur et al., J. Med. Chem. **38** (1995) 3813 und K. Sasaki et al., The Journal of Antibiotics **32** (1979) 849, beschrieben.

Staurosporin ist als Hemmer eines breiten Spektrums von Proteinkinasen bekannt, Geldanamycin hemmt tyrosinspezifische Proteinkinasen und verschiedene andere zelluläre Proteinkinasen wobei die Hemmung der Enzymaktivität direkt oder indirekt erfolgen kann.

Cyclosporin A wirkt als Hemmer der Proteinphosphatase Calcineurin. Darüber hinaus wirkt diese Substanz als Immunsuppressivum und schützt Mitochondrien gegen den durch Noxen ausgelösten Zusammenbruch des Membranpotentials (mitochondrial permeability transition).

Im Fall von Erzeugnissen, die zwei Wirkstoffe enthalten, sind solche Kombinationen bevorzugt, die neben einem Hemmer cyclinabhängiger Proteinkinasen einen Hemmer der Histondeacetylase, einen Hemmer der Proteinbiosynthese, einen Hemmer von Tyrosinkinasen oder insbesondere einen Aktivator streßaktivierter Proteinkinasen enthalten.

Besonders gute Aktivitäten wurden für Erzeugnisse gefunden, die neben dem Hemmer cyclinabhängiger Proteinkinasen mindestens 2, vorzugsweise mindestens 3 weitere Modulatoren proteinmodifizierender Enzyme enthalten. Hierbei sind solche Erzeugnisse bevorzugt, die (a) einen Hemmer cyclinabhängiger Proteinkinasen, einen Hemmer der Proteinbiosynthese und einen Hemmer der Histondeacetylase; (b) einen Hemmer cyclinabhängiger Proteinkinasen, einen Hemmer der Proteinbiosynthese, einen Hemmer der Histondeacetylase und einen Hemmer tyrosinspezifischer Proteinkinasen enthalten. Die genannten Erzeugnisse können jeweils zusätzlich einen Breitband-Proteinkinasehemmer und/oder einen Proteinphosphatasehemmer enthalten.

Besonders bevorzugt sind Erzeugnisse, die neben dem Hemmer cyclinabhängiger Proteinkinasen einen Aktivator streßaktivierter Proteinkinasen und ggf. weitere Modulatoren proteinmodifizierender Enzyme enthalten.

Die Verabreichung des Hemmers cyclinabhängiger Proteinkinasen und der weiteren Modulatoren kann gleichzeitig, d.h. in Form eines Kombinationspräparats, das alle Stoffe enthält, oder getrennt, d.h. durch Gabe von zwei oder mehreren getrennten Präparaten erfolgen. Bei der Gabe von getrennten Präparaten können die Präparate unmittelbar aufeinanderfolgend oder zeitlich abgestuft verabreicht werden. Ein besonders großer Therapieerfolg wird bei zeitlich abgestufter Anwendung erzielt.

Im Fall der besonders bevorzugten Erzeugnisse wird der Hemmer cyclinabhängiger Proteinkinasen vorzugsweise vor dem Aktivator der streßaktivierten Proteinkinasen verabreicht wird. Der zeitliche Abstand zwischen diesen Applikationen hängt von den verwendeten Wirkstoffen ab und beträgt im Fall der bevorzugten Stoffe vorzugsweise etwa 1 bis 12 Stunden, besonders bevorzugt 3 bis 4 Stunden.

Besonders gute Therapieerfolge wurden mit Erzeugnissen erzielt, die zusätzlich zu diesen beiden Wirkstoffen einen Hemmer der Histondeacetylase, einen Hemmer der Proteinbiosynthese oder einen Hemmer von Proteinphosphatasen oder weiterer Proteinkinasen enthalten.

Ganz besonders bevorzugt sind Erzeugnisse, die neben dem Hemmer cyclinabhängiger Proteinkinasen und dem Aktivator streßaktivierter Proteinkinasen zusätzlich 1 bis 4, insbesondere 2 oder 4 Wirkstoffe enthalten, die aus Hemmern der Histondeacetylase, Hemmern der Proteinbiosynthese und Hemmern von Proteinphosphatasen oder Hemmern weiterer Proteinkinasen ausgewählt sind, wobei die zusätzlichen Wirkstoffe vorzugsweise aus unterschiedlichen Wirkstoffgruppen ausgewählt werden.

Beispielhaft für solche Erzeugnisse sind Arzneimittel, die Flavopiridol, Anisomycin, Trichostatin A und Puromycin; oder Flavopiridol, Anisomycin, Trichostatin A, Puromycin, Geldanamycin, Cyclosporin A und ggf. zusätzlich Staurosporin enthalten.

Die erfindungsgemäß verwendeten Wirkstoffe werden vorzugsweise intravenös oder intraperitoneal verabreicht. Die Dosis der Wirkstoffe hängt von dem zu behandelnden Tumor und vom jeweiligen Wirkstoff ab und kann vom Fachmann anhand von Routinetests ermittelt werden. *In vivo* werden die einzelnen Wirkstoffe im allgemeinen in Dosierungen von jeweils 10 nmol bis 1 mmol pro kg Körpergewicht und Therapiezyklus eingesetzt. Diese Applikationsmenge kann in Form einer oder mehrerer Injektionen oder als Infusion verabreicht werden.

Die bevorzugten Wirkstoffe werden vorzugsweise wie folgt dosiert (*in vivo*):

| Wirkstoff | Applikationsdauer*) [h] | Dosierung**) [mg/kg]***) | bevorzugte Dosierung**) [mg/kg]***) |
|---|---|---|---|
| Trichostatin A | 13-42 | 1-100 | 10-50 |
| Puromycin | 13-42 | 1-100 | 10-50 |
| Cyclosporin A | 13-42 | 10-200 | 50-150 |
| Geldanamycin | 13-42 | 0,1-10 | 0,2-6 |
| Flavopiridol | 4-18 | 1-10 | 5-8 |
| Staurosporin | 13-42 | 0,05-1 | 0,1-0,2 |
| Anisomycin | 3-12 | 1-150 | 6-90 |
| Cycloheximid | 13-42 | 0,1-5 | 0,1-2,5 |

*) Gesamtverabreichungsdauer des jeweiligen Wikstoffs
**) über den Applikationszeitraum in Form von 3 bis 6 Bolusinjektionen oder als Infusion zu verabreichende Wirkstoffmenge
***) mg pro kg Körpergewicht

Im allgemeinen sind solche Erzeugnisse bevorzugt, die jeweils 7 µmol bis 40 mmol des Hemmers cyclinabhängiger Proteinkinasen und des oder der Modulatoren proteimodifizierender Enzyme enthalten. Im einzelnen sind Erzeugnisse bevorzugt, die pro Dosierungseinheit die folgenden Wirkstoffmengen enthalten (Lösungen zur Injektion):

| Wirkstoff | Wirkstoffmenge/Dosierungseinheit |
|---|---|
| Trichostatin | 0,7 - 3,5 g/Ampulle |
| Puromycin | 0,7 - 3,5 g/Ampulle |
| Cyclosporin A | 3,5 - 10,5 g/Ampulle |

(fortgesetzt)

| Wirkstoff | Wirkstoffmenge/Dosierungseinheit |
|---|---|
| Geldanamycin | 14 - 420 mg/Ampulle |
| Flavopiridol | 70 - 700 mg/Ampulle |
| Staurosporin | 3,5 - 70 mg/Ampulle |
| Anisomycin | 0,07 - 7 g/Ampulle |
| Cycloheximid | 35 - 170 mg/Ampulle |

Eine Dosierungseinheit/Ampulle enthält die zur Infusion pro Therapiezyklus erforderliche Wirkstoffmenge. Die Dosierungsangaben für die einzelnen Wirkstoffe sind unabhängig davon, in welcher Kombination die Wirkstoffe eingesetzt werden.

Zur Behandlung von Tumorerkrankungen werden die erfindungsgemäßen Arzneimittel vorzugsweise gemäß einem Therapieplan angewendet, der einen oder mehrere mehrstufige, insbesondere dreistufige Therapiezyklen umfaßt.

Die erste, optionale Stufe des Therapiezyklus umfaßt die Gabe von einem oder mehreren Hemmern der Histondeacetylase, vorzugsweise Trichostation A, Hemmern von Tyrosinkinasen, vorzugsweise Geldanamycin, Hemmern der Proteinbiosynthese, vorzugsweise Puromycin, Hemmern von Proteinphosphatasen, vorzugsweise Cyclosporin A und/oder Proteinkinasen, vorzugsweise Staurosporin. Die zusätzliche Applikation der genannten Wirkstoffen der ersten Stufe bewirkt eine deutliche Steigerung der therapeutische Effizienz.

In der zweiten Stufe werden ein oder mehrere Hemmer cyclinabhängiger Kinasen und ggf. Breitbandproteinkinasehemmer eingesetzt.

Die dritte Behandlungsstufe umfaßt die Gabe eines oder mehrerer Aktivatoren stressaktivierter Proteinkinasen und/oder Inhibitoren der Proteinbiosynthese.

Die Gesamtdauer eines Therapiezyklus umfaßt vorzugsweise 14 bis 42 Stunden. Die Applikation von Präparaten der ersten Stufe erstreckt sich dabei vorzugsweise über den gesamten Therapiebereich. Die zweite Behandlungsstufe wird vorzugsweise 12 bis 24 Stunden und die dritte Behandlungsstufe 13 bis 30 Stunden nach Beginn der ersten Stufe begonnen. Die für die einzelnen Stufen charakteristischen Substanzen werden idealerweise jeweils durchgehend bis zum Ende der Gesamttherapie appliziert, so daß sich die Applikation von Präparaten der zweiten und dritten Stufe über einen Zeitraum von 2 bis 18 Stunden bzw. 1 bis 12 Stunden erstreckt. Die zeitliche Abfolge und Dauer der einzelnen Stufen sind in der folgenden Tablle dargestellt. Die Tabelle zeigt auch die Zuordnung der verschiedenen Wirkstoffe zu den Stufen.

**Bevorzugter Therapiezyklus**

**der erfindungsgemäßen Erzeugnisse**

| | <—— 14 - 42 h ——> | | |
|---|---|---|---|
| | <—— 12-24h ——> | <—— 1 - 6h ——> | <—— 1 - 12h ——> |
| 3. Stufe | | | Aktivatoren von SAPKs*) und/oder Inhibitoren der Proteinbiosynthese |
| 2. Stufe | | Inhibitoren cyclinabhängiger Kinasen und ggf. Breitbandproteinkinaseinhibitoren | |
| 1. Stufe | Inhibitoren von Histonsdeacetylasen, Tyrosinkinasen, Proteinphosphatasen und/oder der Proteinbiosynthese und/oder Breitbandproteinkinaseinhibitoren | | |

*) SAPK = stressaktivierte Proteinkinase

Der Therapiezyklus kann mehrfach wiederholt werden, wobei die Zeitintervalle zwischen den einzelnen Therapiezyklen typischerweise 1 bis 14 Tage betragen.

Die Applikation der verschiedenen Wirkstoffe erfolgt vorzugsweise durch mehrfache über den Applikationszeitraum verteilte Bolusinjektionen oder durch kontinuierliche Infusion.

Aufgrund der mehrstufigen Applikationsweise werden die erfindungsgemäßen Erzeugnisse vorzugsweise in Form von 2 bis 4, insbesondere 3 räumlich getrennten Zusammensetzungen hergestellt.

Es wurde überraschend gefunden, daß die Kombination eines Hemmers cyclinabhängiger Proteinkinasen mit einem oder mehreren Modulatoren mindestens eines weiteren proteinmodifizierenden Enzyms eine synergistische und selektive Induktion des Tumorzelltods bewirkt. Bei der systemischen, intravenösen Anwendung an Transplantaten des humanen Adenocarcinoms der Lunge LT23 in der Nacktmaus ließen sich innerhalb von 40 Stunden nach Therapiebeginn 50 bis 90% der behandelten Tumorzellpopulation abtöten (vgl. Beispiel 3). Im Gegensatz dazu bewirken klassische Chemotherapeutika bei Adenokarzinomen in der Regel nur eine Zytostase, d.h. der Zelltod tritt nur in untergeordnetem Maße innerhalb eines Zeitraums von mehreren Tagen oder Wochen ein.

Bei Untersuchungen an Zellkulturen konnte zudem der Tod humaner Tumorzellen u.a. der Blase, der Lunge, des Pankreas, der Brust und des Dickdarms ausgelöst werden.

Die erfindungsgemäßen Kombinationspräparate wirken im Vergleich zu herkömmlichen Tumormitteln außerordentlich schnell, synchron und quantitativ, so daß der behandelten Tumorzellpopulation nur wenig Zeit und Möglichkeit für die Entwicklung von Resistenzen bleibt.

Die Therapie ist erstaunlicherweise kaum von Nebenwirkungen begleitet. In Tierversuchen wurden lediglich kurzfristige, vorübergehende Durchfälle und eine leichte Beeinträchtigung der Spontanaktivität der Versuchstiere beobachtet.

Eine Erklärung für die synergistische Wirkung der erfindungsgemäßen Erzeugnisse und ihre gute Verträglichkeit im Organismus kann gegenwärtig nicht gegeben werden. Die gleichzeitige Gabe eines Hemmers cyclinabhängiger Proteinkinasen und einem oder mehreren Modulatoren mindestens eines weiteren proteinmodifizierenden Enzyms führt offenbar zu Störungen der das Proliferationsverhalten der Zelle steuernden Mechanismen. Normale Zellen reagieren auf solche Störungen gewöhnlich mit einer reversiblen Arretierung des Zellzyklus und versuchen anschließend, die Störung zu kompensieren. Tumorzellen sind, vermutlich infolge ihres veränderten Proliferationsverhaltens, anscheinend nicht in der Lage, einen Zellzyklusarrest korrekt durchzuführen und können somit die durch die erfindungsgemäßen Erzeugnisse hervorgerufenen Störungen schlechter kompensieren. Die trotz Störung fortgesetzte Proliferation führt wahrscheinlich zur Auslösung des Zelltodesprogramms.

Die Herstellung pharmazeutischer Zusammensetzungen und Zubereitungen erfolgt in an sich bekannter Weise indem die Wirkstoffe gemeinsam oder getrennt zusammen mit üblichen pharmazeutischen Träger-, Verdünnungs- und/oder Hilfsstoffen vermischt und diese Mischungen zu Tabletten, Kapseln oder vorzugsweise Lösungen für die intravenöse oder intraperitoneale Injektion oder Infusion geformt werden.

Bevorzugt sind Erzeugnisse, bei denen die Einzelwirkstoffe jeweils in separaten Formulierungen vorliegen, so daß eine getrennte oder zeitlich abgestufte Verabreichung möglich ist. Die Formulierungen sind aufeinander abgestimmt, d.h. die Konzentrationen der einzelnen Wirkstoffe der Kombination sind so gering, daß Nebenwirkungen fast vollständig ausgeschlossen werden und die Einzelsubstanzen allein keine oder zumindest keine ausreichende Zelltod-induzierende Wirkung aufweisen. Erst durch die Kombination mit den anderen genannten Wirkstoffen wird eine effiziente Tumortherapie möglich.

Im folgenden wird die Erfindung anhand von Ausführungsbeispielen näher erläutert.

## Beispiel 1

### Synergistische Induktion des Tumorzelltods in vitro durch Kombination eines Hemmers cyclinabhängiger Kinasen mit Modulatoren proteinmodifizierender Enzyme (Zwei- und Dreistoffkombinationen)

Die dargestellten Ergebnisse wurden mit der aus einem humanen Adenokarzinom der Lunge gewonnenen adhärent in Gewebekultur wachsenden Zellinie LT23 erhalten. Alle *in vitro* Experimente wurden in Zellkulturinkubatoren bei 37 °C in einer wasserdampfgesättigten Atmosphäre von 5% $CO_2$ in Luft durchgeführt. Der pH-Wert des Kulturmediums lag in den Vorratskulturen und bei den in den Abbildungen 1 bis 4 dargestellten Experimenten zwischen 7,2 und 7,4.

Vorratskulturen der LT23 Zellinie wurden montags und freitags regelmäßig in kollagenbeschichteten 58 $cm^2$ Gewebekulturschalen (Fa. Greiner) in serumfreiem Kulturmedium (KM1) passagiert. Für die Kollagenbeschichtung wurden die Kulturschalen mit jeweils 5 ml einer 1:100-Verdünnung von solubilisiertem Kalbshautkollagen (Fa. Böttger, Berlin, Kat.-Nr. 9004 B) in Basalmedium (BM) für 4 h bei 37 °C inkubiert. Danach wurde die kollagenhaltige Lösung entfernt und bis zur Aussaat der Zellen durch 10 ml BM ersetzt. Für die Passage wurden die Zellen von Vorratskulturen durch Behandlung (5 min, 37 °C) mit einer Trypsinlösung (Boehringer Mannheim, Kat.-Nr. 109827, 20 µg/ml in NaCl/HEPES*) in eine Einzelzellsuspension überführt, nachdem die Vorratskulturen zuvor zweimal mit je 10 ml NaCl/HEPES gewaschen worden waren. Nach Zentrifugation der Einzelzellsuspension wurden die Zellen in KM1 resuspendiert, an einem elektronischen Partikelzählgerät (Coulter Counter) gezählt und in einer Zelldichte von 0,5 x $10^6$ oder 1 x $10^6$ pro Kulturschale in 15 ml Medium ausgesät.

Für die Versuche wurden LT23-Kulturen mittels Trypsin wie oben beschrieben in Einzelzellsuspensionen überführt und 200 000 Zellen pro Loch auf kollagenbeschichtete 24-Loch-Gewebekulturplatten (Firma NUNC) in jeweils 1 ml KM2 ausgesät. 8 Stunden nach der Aussaat wurden die folgenden Substanzen als Konzentrate in jeweils 0,1 ml KM2 pro Loch zugesetzt (Zeitpunkt t = 0 h), so daß die angegebenen Konzentrationen in den Kulturen erhalten wurden:

K: Kontrolle ohne Substanz,
C: Cyclosporin A (Sandoz, Nürnberg, 1,5 µg/ml),
S: Staurosporin (Serva, Boehringer Ingelheim KG, Kat.-Nr. 35385, 20 nM),
T: Trichostatin A (Wako, Neuss, Kat.-Nr. B20411991, 150 ng/ml),
X: Cycloheximid (Sigma, Deisenhofen, Kat.-Nr. C7698, 1 µg/ml),
P: Puromycin (Serva, Boehringer Ingelheim KG, Kat.-Nr. 33835, 500 ng/ml),
E: Emetin (Sigma, Deisenhofen, Kat.-Nr. E 2375, 300 ng/ml),
A: Anisomycin (Sigma, Deisenhofen, Kat.-Nr. A 9789, 150 ng/ml),
G: Geldanamycin (Calbiochem, Bad Soden, Kat.-Nr. 345805, 100 ng/ml).
F: Flavopiridol (National Cancer Institute, Drug Synthesis & Chemistry Branch, Bethesda, USA, 400 ng/ml) wurde erst zum Zeitpunkt t = 16 h zugesetzt.

Zum Zeitpunkt t = 22 h wurden die substanzhaltigen Medien quantitativ von den Kulturen entfernt und durch substanzfreies Kontrollmedium ersetzt.

Zu den Zeitpunkten t = 16 h und t = 40 h wurden von jeder Therapiekondition jeweils 3 Replikate bezüglich der Anzahl vitaler Zellen evaluiert. Die Anzahl der vitalen Zellen in Prozent der Zellzahl der unbehandelten Ausgangskultur

* HEPES = N-(2-Hydroxyethyl)piperazin-N'-2-ethansulfonsäure (Serva, Boehringer Ingelheim KG, Kat.-Nr. 25245)
NaCl/HEPES: 0,9 % NaCl/10 mM HEPES pH 7,4

zum Zeitpunkt t = 0 h ist in Abbildung 1 graphisch dargestellt. Innerhalb einer Gruppe von 3 Replikaten betrug die Abweichung vom jeweiligen Mittelwert 2 bis 10 %. In den Abbildungen geben die oben links eingetragenen waagerechten Balken den Expositionszeitraum für die über dem Balken bezeichneten Substanzen an.

Aus der Abbildung 1 geht hervor, daß die verwendeten therapeutischen Substanzen in den eingesetzten Konzentrationen als Einzelsubstanzen lediglich eine wachstumshemmende Wirkung haben.

In einem zweiten Experiment wurde wie oben beschrieben verfahren, jedoch wurden alle Kulturen bis auf die Kontrolle zum Zeitpunkt t = 16 h mit Flavopiridol (F) in der oben angegebenen Menge versetzt. Die Ergebnisse der Zweistoffkombinationen sind in Abbildung 2 graphisch dargestellt. Die 2-Substanz-Kombinationen mit Flavopiridol als festem Bestandteil bewirken zwischen t = 16 h und t = 40 h eine signifikante Nettoabhahme der Zahl vitaler Tumorzellen in den behandelten Kulturen. Im Vergleich zur Ausgangskultur wurde die Zellzahl um bis zu 50 % gesenkt. Im gleichen Zeitraum stieg die Zellzahl in den unbehandelten Kontrollkulturen (K) von 100 % auf 300 % an, so daß die Anzahl der vitalen Zellen in den behandelten Kulturen zum Zeitpunkt t = 40 h nur etwa 15 bis 30 % der Zellzahl in der unbehandelten Kontrollkultur betrug.

In einem weiteren Versuch wurden alle Zellkulturen mit Ausnahme von K und F zum Zeitpunkt t = 19 h zusätzlich mit Anisomycin in der oben angegebenen Menge versetzt. Hierdurch wurde eine weitere, deutliche Aktivitätssteigerung erzielt und es konnten bis zu 99 % der ursprünglichen Tumorzellen abgetötet werden (Abb. 3).

Ähnliche Ergebnisse wurden erzielt, indem die Zweistoffgemische zum Zeitpunkt t = 0 mit Trichostatin (A) (150 ng/ml) versetzt wurden (Abb. 4), oder durch Verwendung von Dreistoffkombinationen auf Basis von Puromycin und Flavopiridol oder Geldanamycin und Flavopiridol.

In den Versuchen wurden die folgenden Kulturmedien eingesetzt:

BM 1:1 Mischung aus Dulbecco's modified Eagle's medium (DMEM) und Ham's F12 nutrient mixture (beide von Gibco/BRL), gepuffert mit 22 mM $NaHCO_3$ und 15 mM HEPES.

KM1 KM1 wurde aus Basalmedium (BM) durch Supplementierung mit Insulin aus Rinderpankreas (Sigma, Deisenhofen, Kat.-Nr. I 5500, 5 µg/ml), Transferrin (human, Sigma, Kat.-Nr. T 2252, 5 µg/ml), Epidermal Growth Factor (EGF, human, Fa. TEBU, Frankfurt, Kat.-Nr. 100-15, 5 ng/ml), Trijodthyronin (Sigma, Kat.-Nr. T 6397, 0,1 nM), Na-Selenit (Sigma, Kat.-Nr. S 1382, 75 nM), Ethanolamin (Sigma, Kat.-Nr. E 0135, 8 µM), Ölsäure (Sigma, Kat.-Nr. O 1383, 2,5 µg/ml) und fettsäurefreiem Rinderserumalbumin (Sigma, Kat.-Nr. A 6003) hergestellt.

KM2 wird aus KM1 durch Zusatz von Rinderserumalbumin (Serva, Boehringer Ingelheim KG, Kat.-Nr. 11930, 20 mg/ml) hergestellt.

Zur Bestimmung des Anteils vitaler Zellen wurde das Medium aus den Kulturlöchern durch Absaugen entfernt und durch 1,5 ml einer 0,25 %igen Triton-X-100-Lösung (Serva, Boehringer Ingelheim KG, Kat.-Nr. 37240) in 0,4 mM Ethylendiamintetraessigsäure (EDTA, pH 7,5) ersetzt. Unter diesen Bedingungen werden die Zellmembranen der Tumorzellen aufgelöst, während die Zellkerne schwellen und freigesetzt werden, so daß sich eine Einzelkernsuspension bildet. Nach 10 min wurde die Kernsuspension durch Zusatz von 0,5 ml einer 10 %igen Formaldehydlösung fixiert und mittels Coulter Counter (Coulter Counter Typ ZM mit angeschlossener Größenverteilungsanalyse Coulter Channelyzer 256) die Gesamtpartikelzahl (G) bestimmt. Durch die Triton-Behandlung werden nur die Kerne vitaler Zellen freigesetzt, während tote Zellen aufgrund von Proteinquervernetzungen von Triton-X-100 nicht desintegriert werden, also keinen Kern freisetzen und als "sklerotisierte" Zellkörper erhalten bleiben. Die relativen Anteile intakter Kerne (K) und toter Zellkörper (T) an der Gesamtpartikelzahl (G) können in der Durchflußzytometrie (Coulter Durchflußcytometer Epics XL) aufgrund der unterschiedlichen Seitwärtslichtstreusignale der Partikelpopulationen bestimmt werden (vgl. J. Breder, S. Rüller, E. Rüller, M. Schlaak, J. van der Bosch, Exp. Cell Res. **223** (1996) 259-267). Die Anzahl vitaler Zellen (V) ergibt sich wie folgt: $V = G \times K/(K+T)$ .

**Beispiel 2**

**Synergistische Induktion des Tumorzelltods <u>in vitro</u> durch Kombination eines Hemmers cyclinabhängiger Kinasen mit Modulatoren proteinmodifizierender Enzyme (Mehrstoffkombinationen)**

Analog zu Beispiel 1 wurden Kombinationen von 4 oder 5 Substanzen auf der Basis von Geldanamycin (G), Flavopiridol (F) und Anisomycin (A) getestet. Abweichend von Beispiel 1 wurden die LT23-Zellkulturen 8 Stunden vor dem Zeitpunkt t = 0 h mit 100 µl 0,12 N Salzsäure versetzt, um den pH-Wert von 7,4 auf 6,8 zu senken. Unter diesen Bedingungen sind die Zellkulturen weniger therapiesuszeptibel, so daß auch Substanzkombinationen, die bei pH 7,4 die Tumorzellen zu 100 % abtöten, quantitativ hinsichtlich ihres relativen Wirkstoffpotentials bewertet werden können.

Abbildung 5 zeigt, daß unter diesen Bedingugen die Zahl der Tumorzellen trotz Zugabe von Flavopiridol weiter

ansteigt. Durch die zum Vergleich mitgetestete Dreistoffkombination GFA konnten ca. 90 % der Tumorzellen abgetötet werden, während durch die untersuchten Vier- und Fünfstoffkombinationen bis zu ca. 99 % der ursprünglichen Zellen abgetötet wurden.

**Beispiel 3**

**Induktion des in vivo Zelltods von humanen Adenokarzinomzellen der Lunge**

1,5 × $10^6$ Zellen eines humanen Adenokarzinoms der Lunge wurde pro Maus in 100 µl Kulturmedium subkutan zwischen den Schulterblättern inokuliert. 12 Tage nach Inokulation hatten sich etwa erbsengroße Tumorknoten gebildet. Zu diesem Zeitpunkt wurde die Therapie begonnen (t = 0 Stunden). Hierzu wurden jeweils 150 µl einer Puromycinlösung und 400 µl einer Trichostatinlösung zu den Zeiten 0 Stunden, 8 Stunden und 16 Stunden in die Schwanzvene injiziert. Zum Zeitpunkt 16 Stunden wurden 100 µl Flavopiridollösung und im Zeitraum 19 bis 20 Stunden wurden im Abstand von 20 min 150 µl, 100 µl, 50 µl bzw. 50 µl Anisomycinlösung in die Schwanzvene appliziert. Die Tiere wurden zum Zeitpunkt 40 Stunden zur Entnahme der Tumoren getötet. Die entnommenen Tumoren wurden umgehend mittels Skalpell halbiert und in 10%iger Formalinlösung für die Histologie fixiert.

Es wurden folgende Lösungen eingesetzt:

| | |
|---|---|
| Puromycin: | 667 µg/ml, in Wasser enthaltend 0,9% NaCl/0,24% BSA/10 mM HEPES/pH 7,4 |
| Trichostatin A: | 500 µg/ml, in Wasser enthaltend 0,9% NaCl/4,5% BSA/1,25% DMSO/10 mM HEPES/pH 7,4 |
| Flavopiridol: | 5 mM, in Wasser enthaltend 0,9% NaCl/0,24% BSA/10% DMSO/10 mM HEPES/pH 7,4 |
| Anisomycin: | 6 mg/ml, in Wasser enthaltend 0,9% NaCl/0,24% BSA/6% DMSO/10 mM HEPES/pH 7,4 |

Zur Herstellung der Lösungen wurden Konzentrate von Puromycin (25 mg/ml Wasser), Trichostatin A (40 mg/ml DMSO), Flavopiridol (50 mM in DMSO) und Anisomycin (100 mg/ml DMSO) verwendet.

| | |
|---|---|
| BSA: | Bovines Serumalbumin |
| DMSO: | Dimethylsulfoxid |
| HEPES: | N-(2-Hydroxyethyl)piperazin-N'-2-ethansulfonsäure |

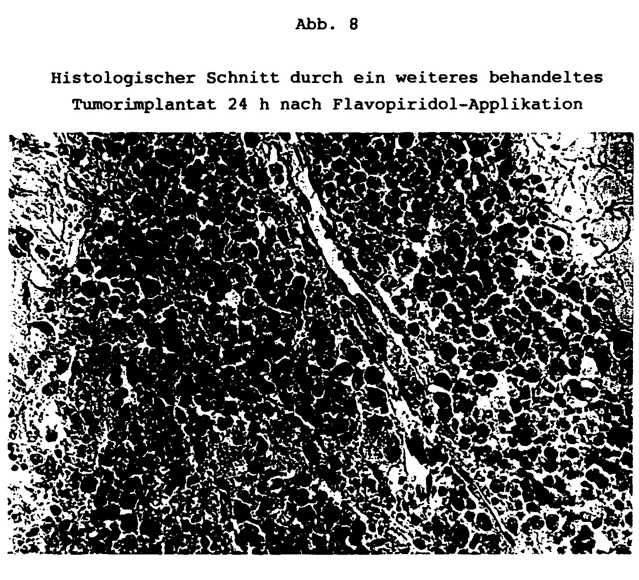

Abbildung 6 Zeigt einen histologischen Schnitt eines unbehandelten Tumortransplantats und Abbildungen 7 und 8 Schnitte von Zwei unterschiedlich behandelten Tumortransplantaten 24 Stunden nach der Flavopiridol-Applikation. Die massenhafte Induktion von dunkel gefärbten Pyknosen (tote Zellen) in den behandelten Tumortransplantaten ist offensichtlich.

**Patentansprüche**

1. Erzeugnisse, enthaltend einen Hemmer cyclinabhängiger Proteinkinasen und einen oder mehrere Modulatoren mindestens eines weiteren proteinmodifizierenden Enzyms als Kombinationspräparat zur gleichzeitigen, getrennten oder zeitlich abgestuften Anwendung als Arzneimittel, wobei Zweistoffgemische aus Flavopiridol und Cycloheximid ausgenommen sind.

2. Erzeugnis gemäß Anspruch 1, **dadurch gekennzeichnet**, daß es als Hemmer cyclinabhängiger Proteinkinasen ein 4H-1-Benzopyran-4-on-Derivat gemäß der Formel

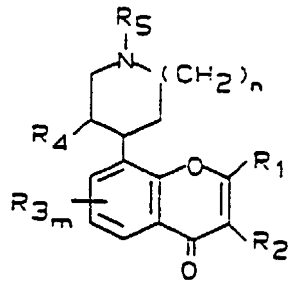

enthält, in der

- $R^1$ Wasserstoff, $C_1$-$C_6$-Alkyl, Aryl-$C_1$-$C_4$-alkyl, substituiertes $C_1$-$C_6$-Alkyl, $C_{3\text{-}6}$-Cycloalkyl, einen $C_3$-$C_9$-Heterozyklus mit 1, 2 oder 3 Heteroatomen wie N, S, O oder einer beliebigen Kombinationen dieser Atome, $C_3$-$C_6$-Cycloalkyl-$C_1$-$C_4$-alkyl, $C_2$-$C_6$-Alkenyl, $C_2$-$C_6$-Alkinyl, Aryl (einschließlich polycyclischer Ringe), einen aromatischen heterocyclischen Rest, substituiertes Aryl, -COOH, eine Aldehyd-$C_1$-$C_4$-alkylgruppe, eine COO-$C_1$-$C_4$-alkylgruppe, eine primäre Amino-, Alkylamino-, Aralkylamino-, Dialkylamino-, Amido-, Arylamino-, Diarylaminogruppe oder -$CH_2O$-$C_1$-$C_4$-Alkyl bedeutet;
- $R^2$ Wasserstoff oder $C_1$-$C_6$-Alkyl, Aryl, Nitro, Amino, Di-$C_1$-$C_4$-alkylamino, Halogen, Hydroxy, Alkoxy, -COOH, -COO-$C_1$-$C_4$-Alkyl, -CHO, -$CH_2OH$ oder -$CH_2O$-$C_1$-$C_4$-Alkyl bedeutet;
- $R^3$ Wasserstoff oder $C_1$-$C_4$-Alkyl, substituiertes $C_1$-$C_4$-Alkyl, Hydroxy, -COOH, Nitro, eine Amino-, $C_1$-$C_4$-Alkylamino-, Di-$C_1$-$C_4$-alkylaminogruppe, Halogen, O-Alkyl-C(=0)-alkyl, -CHO, -$CH_2OH$, -$CH_2O$-$C_1$-$C_4$-Alkyl, $R_2N$-C(=0)-O- bedeutet, wobei
- R H, $C_1$-$C_6$-Alkyl, Cycloalkyl O-Alkyl-C(=O)-alkyl oder Aryl ist;
- $R^4$ Wasserstoff, Hydroxy, $C_1$-$C_4$-Alkoxy, $C_1$-$C_4$-Alkanoyloxy, $C_1$-$C_4$-Alkoxycarbonyl, Aryloxy, eine Amino-, $C_1$-$C_4$-Alkylamino-, Di-$C_1$-$C_4$-alkylaminogruppe, $R_2N$-C(=O)-O-, bedeutet und R die oben angegebene Bedeutung hat;
- $R^5$ Wasserstoff, $C_1$-$C_6$-Alkyl, Aryl-$C_1$-$C_4$-alkyl, $C_3$-$C_6$-Cycloalkyl, $C_3$-$C_6$-Cycloalkyl-$C_1$-$C_4$-alkyl, Alkylamino-, $C_1$-$C_4$-Alkanoyl, -C(=O)-O-$C_1$-$C_4$-Alkyl oder Aroyl bedeutet, wobei die Arylgruppe ein unsubstituierter oder mono- oder polysubstituierter Phenylrest ist;
- m eine ganze Zahl zwischen 0 und 3; und
- n eine ganze Zahl zwischen 0 und 2 ist.

3. Erzeugnis gemäß Anspruch 2, **dadurch gekennzeichnet**, daß

- $R^1$ H, $C_1$-$C_3$-Alkyl, Naphthyl, Aryl, subsituiertes Aryl, Aralkyl oder ein aliphatischer oder aromatischer $C_3$-$C_9$-Heterocyclus mit 1, 2 oder 3 Heteroatomen wie N, S, O oder einer beliebigen Kombination dieser Atome;
- $R^2$ H oder $C_1$-$C_3$-Alkyl;
- $R^3$ OH oder $OCH_3$;
- $R^4$ OH;
- $R^5$ $C_1$-$C_3$-Alkyl, $C_3$-$C_5$-Cycloalkyl, $C_3$-$C_5$-Cycloalkyl-$C_1$-$C_4$-Alkyl;
- m = 1 oder 2; und/oder
- n = 1 ist.

4. Erzeugnis gemäß Anspruch 3, **dadurch gekennzeichnet**, daß es als Hemmer cyclinabhängiger Proteinkinasen Flavopiridol enthält.

5. Erzeugnis gemäß einem der Ansprüche 1 bis 4, **dadurch gekennzeichnet**, daß es als Modulator proteinmodifizierender Enzyme einen Aktivator streßaktivierter Proteinkinasen enthält.

6. Erzeugnis gemäß Anspruch 5, **dadurch gekennzeichnet**, daß es Anisomycin oder ein Anisomycin-Derivat gemäß der Formel

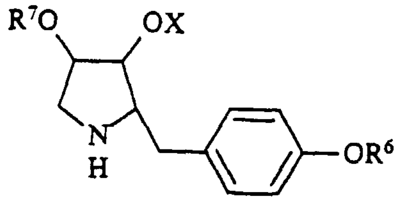

enthält, in der

| | |
|---|---|
| $R^6$ | Wasserstoff oder $C_1$-$C_6$-Alkyl bedeutet; |
| $R^7$ | Wasserstoff oder eine gesättigte oder ungesättigte, lineare, verzweigte oder cyclische $C_1$-$C_{18}$-Acylgruppe bedeutet; und |
| X | $C_1$-$C_6$-Alkyl-CO-; -CO-$NR^8R^9$ oder -$CH_2R^{10}$ bedeutet, wobei |
| $R^8$ und $R^9$ | unabhängig voneinander Wasserstoff, eine lineare oder cyclische $C_1$-$C_6$-Alkylgruppe, Phenyl, eine substituierte lineare oder verzweigte $C_1$-$C_6$-Alkylgruppe oder einen substituierten Phenylrest bedeuten; und |
| $R^{10}$ | Wasserstoff, $C_1$-$C_6$-Alkyl, $C_1$-$C_6$-Alkoxy oder eine substituierte $C_1$-$C_6$-Alkoxygruppe bedeutet. |

**7.** Erzeugnis gemäß Anspruch 6, **dadurch gekennzeichnet**, daß

$R^6$ $CH_3$;
$R^7$ $C_1$-$C_{12}$-Acyl
$R^8$ und $R^9$ H;
$R^{10}$ H oder $C_{1\text{-}3}$-Alkyl bedeutet und
X gleich -C(=O)$NH_2$, $C_1$-$C_6$-Alkyl-C(=O)- ist.

**8.** Erzeugnis gemäß Anspruch 7, **dadurch gekennzeichnet**, daß es als Aktivator für streßaktivierte Proteinkinasen Anisomycin enthält.

**9.** Erzeugnis gemäß einem der Ansprüche 5 bis 8 zur Therapie durch Applikation des Hemmers für cyclinabhängige Proteinkinasen, gefolgt von der Applikation des Aktivators für streßaktivierte Proteinkinasen im zeitlichen Abstand von 1 bis 12 Stunden.

**10.** Erzeugnis gemäß einem der Ansprüche 1 bis 9, **dadurch gekennzeichnet**, daß es als Modulator proteinmodifizierender Enzyme einen Hemmer der Histondeacetylase enthält.

**11.** Erzeugnis gemäß Anspruch 10, **dadurch gekennzeichnet**, daß es als Hemmer der Histondeacetylase ein pharmazeutisch verträgliches Salz der Buttersäure, Trichostatin A, Trapoxin, Chlamydocin, HC-Toxin, WP-3161 und/oder Cyt 2 enthält.

**12.** Erzeugnis gemäß einem der Ansprüche 1 bis 11, **dadurch gekennzeichnet**, daß es als Modulator proteinmodifizierender Enzyme einen Hemmer der Proteinbiosynthese enthält.

**13.** Erzeugnis gemäß Anspruch 12, **dadurch gekennzeichnet**, daß es als Hemmer der Proteinbiosynthese Puromycin, Cycloheximid und/oder Emetin enthält.

**14.** Erzeugnis gemäß einem der Ansprüche 1 bis 13, **dadurch gekennzeichnet**, daß es als Modulator proteinmodifizierender Enzyme einen Hemmer von Proteinphosphatasen oder weiterer Proteinkinasen enthält.

**15.** Erzeugnis gemäß Anspruch 14, **dadurch gekennzeichnet**, daß es Staurosporin, ein Staurosporin-Derivat, Cyclosporin A, Geldanamycin, Dihydrogeldanamycin, ein Geldanamycin- und/oder Dihydrogeldanamycin-Derivat gemäß der Formel

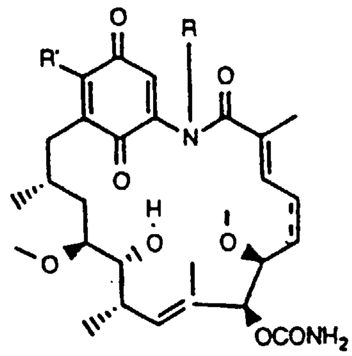

enthält, in der

R H ist und

R' eine primäre, sekundäre oder tertiäre Aminogruppe, wie eine einfach oder zweifach durch $C_1$-$C_6$-Alkyl- oder $C_1$-$C_6$-Alkenylreste substituierte Aminogruppe, insbesondere $-NH_2$, $-NH-(CH_2)_n-CH_3$,

$$-N<\begin{matrix}(CH_2)_m-CH_3\\(CH_2)_n-CH_3\end{matrix}\quad,\quad -N<\begin{matrix}CH_2\\|\\(CH_2)_p\end{matrix}\quad \text{oder}\quad -NH-CH<\begin{matrix}CH_2\\|\\(CH_2)_p\end{matrix}$$

bedeutet, wobei n und m unabhängig voneinander 0 bis 6 und p 1 bis 7 sind.

**16.** Erzeugnis gemäß einem der Ansprüche 1 bis 15, **dadurch gekennzeichnet**, daß es jeweils 7 µmol bis 40 mmol des Hemmers cyclinabhängiger Proteinkinasen und des oder der Modulatoren proteinmodifizierender Enzyme enthält.

**17.** Erzeugnis gemäß einem der Ansprüche 1 bis 16 zur intravenösen Applikation.

**18.** Erzeugnis gemäß einem der Ansprüche 1 bis 17 zur Therapie durch ein- bis viermalige oder kontinuierliche Applikation von (a) ggf. einem oder mehreren Histondeacetylasehemmern, Hemmern der Proteinbiosynthese, Hemmern von Proteinphosphatasen und/oder Hemmern von weiteren Proteinkinasen, (b) einem oder mehreren Hemmern cyclinabhängiger Proteinkinasen und ggf. Breitband-Proteinkinasehemmern sowie (c) einem oder mehreren Aktivatoren für streßaktivierte Proteinkinasen und ggf. Hemmern der Proteinbiosynthese.

**19.** Erzeugnis gemäß Anspruch 18 zur Applikation des oder der Wirkstoffe gemäß (a) über einen Zeitraum von 14 bis 42 Stunden, des oder der Wirkstoffe gemäß (b) über einen Zeitraum von 2 bis 13 Stunden und des oder der Wirkstoffe gemäß (c) über einen Zeitraum von 1 bis 12 Stunden.

**20.** Erzeugnis gemäß Anspruch 19, zur Applikation des oder der Wirkstoffe gemäß (b) im zeitlichen Abstand von 12 bis 24 Stunden nach Beginn der Applikation des oder der Wirkstoffe gemäß (a) und des oder der Wirkstoffe gemäß (c) im zeitlichen Abstand von 1 bis 6 Stunden nach Beginn der Applikation des oder der Wirkstoffe gemäß (b).

**21.** Verfahren zur Herstellung eines Erzeugnisses gemäß den Ansprüchen 1 bis 20, **dadurch gekennzeichnet**, daß man mindestens einen Hemmer cyclinabhängiger Proteinkinasen und mindestens einen Modulator proteinmodifizierender Enzyme mit üblichen pharmazeutischen Träger-, Verdünnungs- und/oder Hilfsstoffen, getrennt oder zusammen vermischt und diese Mischung(en) Zu Tabletten, Kapseln oder Lösungen für die lokale, orale, parenterale, insbesondere intravenöse oder intraperitoneale Injektion oder Infusion formt.

**22.** Verwendung eines Hemmers cyclinabhängiger Proteinkinasen und eines Modulators proteinmodifizierender Enzyme zur Herstellung von Arzneimittelzubereitungen.

**23.** Verwendung nach Anspruch 22 zur Herstellung von Arzneimittelzubereitungen zur Behandlung von Tumoren.

**24.** Verwendung gemäß einem der Ansprüche 22 bis 23 zur Behandlung von Tumoren der Blase, Lunge, Brust, des Pankreas und Dickdarms.

**25.** Verwendung nach einem der Ansprüche 20 bis 22 zur Herstellung von Arzneimittelzubereitungen zur intravenösen Therapie.

**26.** Verwendung nach einem der Ansprüche 22 bis 25 zur Herstellung von Arzneimittelzubereitungen zur einfachen, mehrfachen oder kontinuierlichen Applikation von (a) ggf. einem oder mehreren Histondeacetylasehemmern, Hemmern der Proteinbiosynthese, Hemmern von Proteinphosphatasen und/oder Hemmern von weiteren Proteinkinasen (b) einem oder mehreren Hemmern für cyclinabhängige Kinasen und ggf. Breitband-Proteinkinasehemmern und (c) einem oder mehreren Aktivatoren für streßaktivierte Proteinkinasen und ggf. Hemmern der Proteinbiosynthese.

**27.** Verwendung nach Anspruch 26 zur Applikation des oder der Wirkstoffe gemäß (a) über einen Zeitraum von 12 bis 42 Stunden, des oder der Wirkstoffe gemäß (b) über einen Zeitraum von 1 bis 18 Stunden und des oder der Wirkstoffe gemäß (c) über einen Zeitraum von 1 bis 12 Stunden.

**28.** Verwendung eines Hemmers cyclinabhängiger Proteinkinasen und eines oder mehrerer Modulatoren mindestens eines weiteren proteinmodifizierenden Enzyms in Form eines aus mindestens zwei räumlich getrennten Zusammensetzungen, von denen die eine den Hemmer cyclinabhängiger Proteinkinasen (Zusammensetzung A) und die andere den oder die Modulatoren mindestens eines weiteren proteinmodifizierenden Enzyms (Zusammensetzung B) enthält, bestehenden Mittels zur aufeinanderfolgenden und/oder gleichzeitigen Anwendung bei der Behandlung von Tumoren bei Menschen oder Säugetieren nach einem Therapieplan, der aus einem oder mehreren Zyklen besteht, wobei jeder Therapiezyklus folgende Stufen umfaßt:

Stufe 1 (optional) einfache, mehrfache oder kontinuierliche Applikation der Zusammensetzung (B);
Stufe 2 einfache, mehrfache oder kontinuierliche Applikation der Zusammensetzung (A);
Stufe 3 einfache, mehrfache oder kontinuierliche Applikation der Zusammensetzung (B).

**29.** Verwendung nach Anspruch 28, **dadurch gekennzeichnet**, daß Stufe 1 die Applikation eines oder mehrerer Histondeacetylasehemmer, Hemmer der Proteinbiosynthese, Hemmer von Proteinphosphatasen und/oder von Hemmern von weiteren Proteinkinasen und Stufe 3 die Applikation eines oder mehrerer Aktivatoren für streßaktivierte Proteinkinasen und ggf. Hemmern der Proteinbiosynthese umfaßt.

**30.** Verwendung nach Anspruch 29, **dadurch gekennzeichnet**, daß Stufe 2 zusätzlich die Applikation eines Breitband-Proteinkinasehemmers umfaßt.

**31.** Verwendung nach einem der Ansprüche 28 bis 30, **dadurch gekennzeichnet**, daß sich Stufe 1 über einen Zeitraum von 12 bis 42 Stunden, Stufe 2 über einen Zeitraum von 1 bis 18 Stunden und Stufe 3 über einen Zeitraum von 1 bis 12 Stunden erstreckt.

**32.** Arzneimittel, enthaltend einen Hemmer cyclinabhängiger Proteinkinasen und einen oder mehrere Modulatoren mindestens eines weiteren proteinmodifizierenden Enzyms als Kombinationspräparat zur gleichzeitigen, getrennten oder zeitlich abgestellten Anwendung.

**Abb. 1:**
**Einzelsubstanzen**

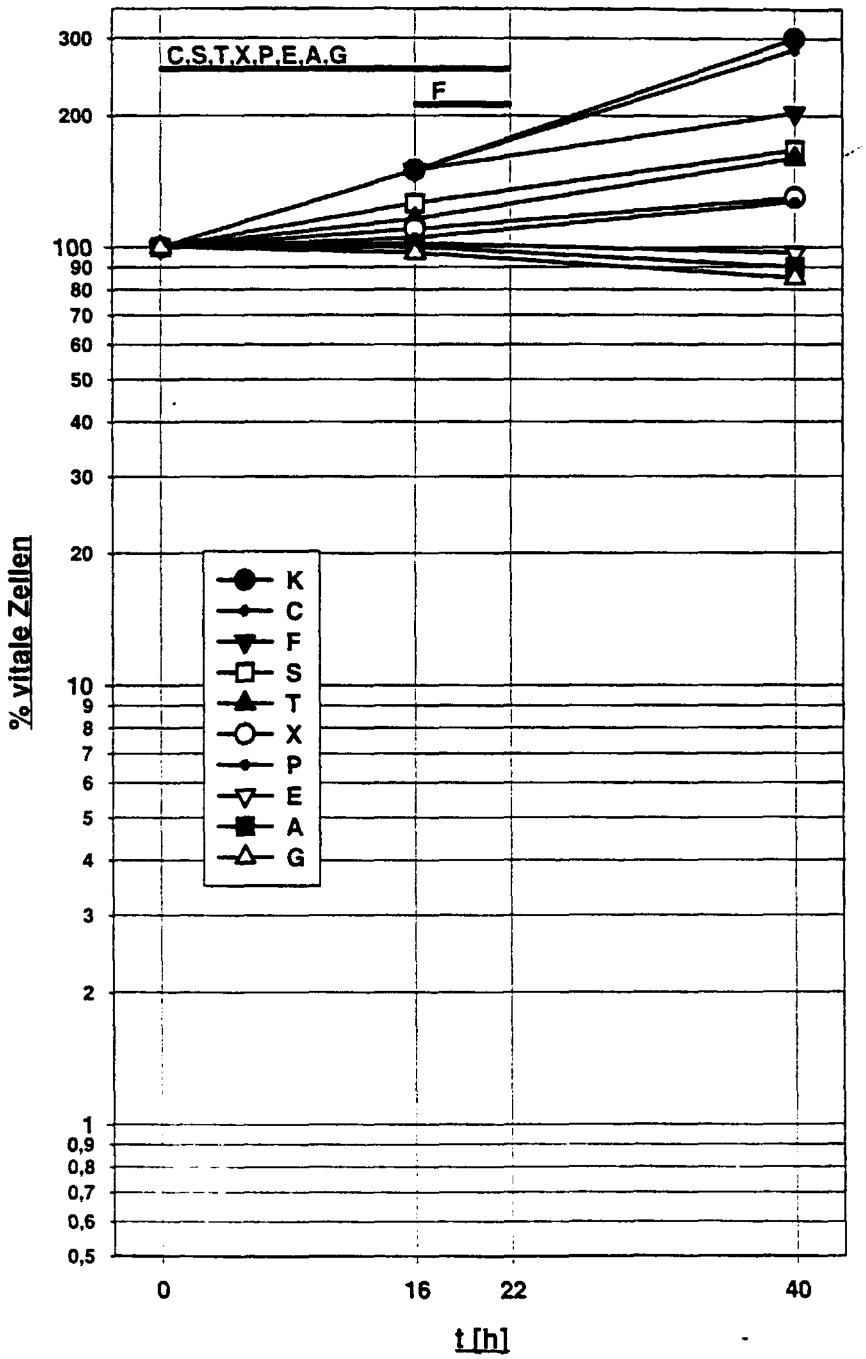

Abb. 2:
Kombinationen von 2 Substanzen
auf der Basis F

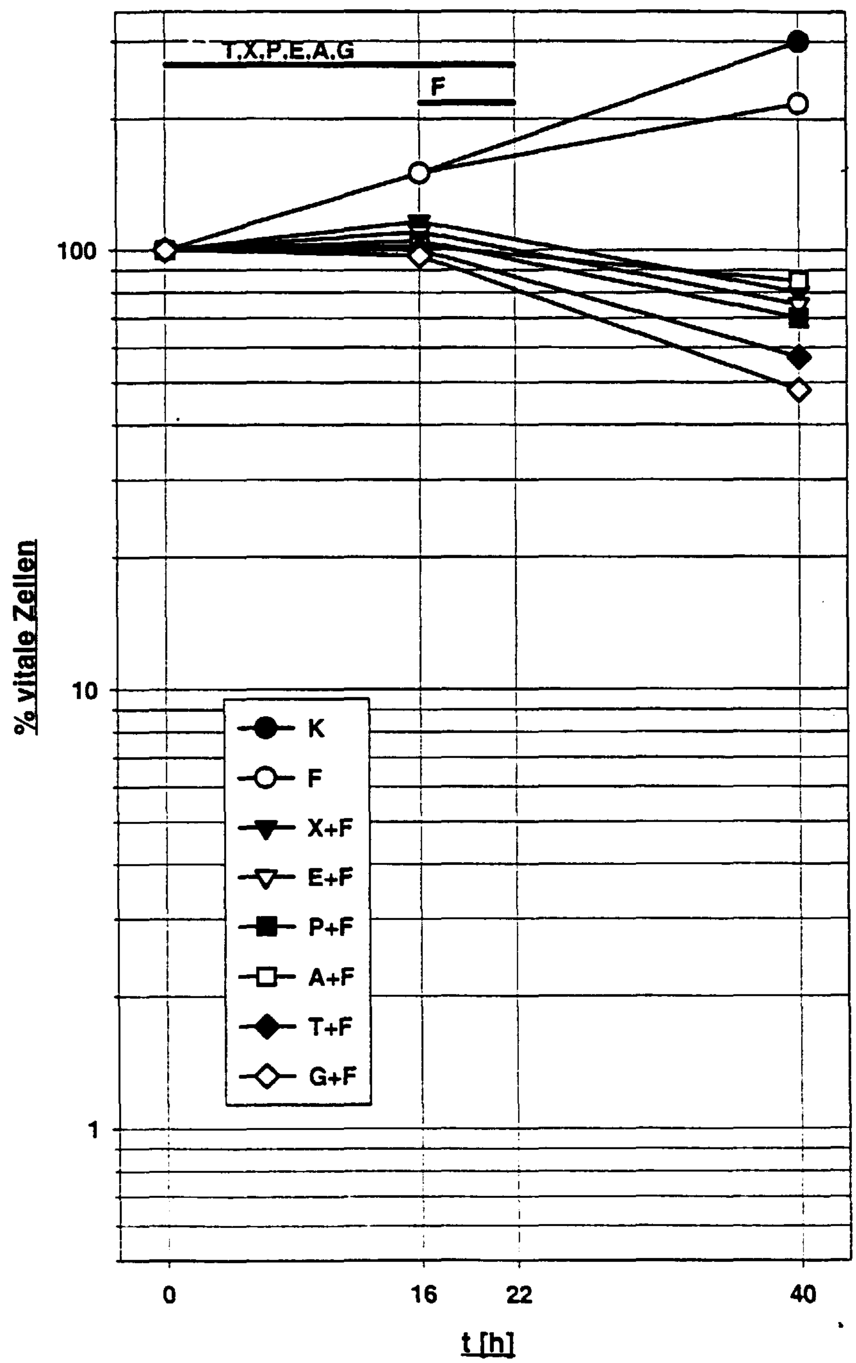

Abb. 3:
Kombinationen von 3 Substanzen
auf der Basis F+A

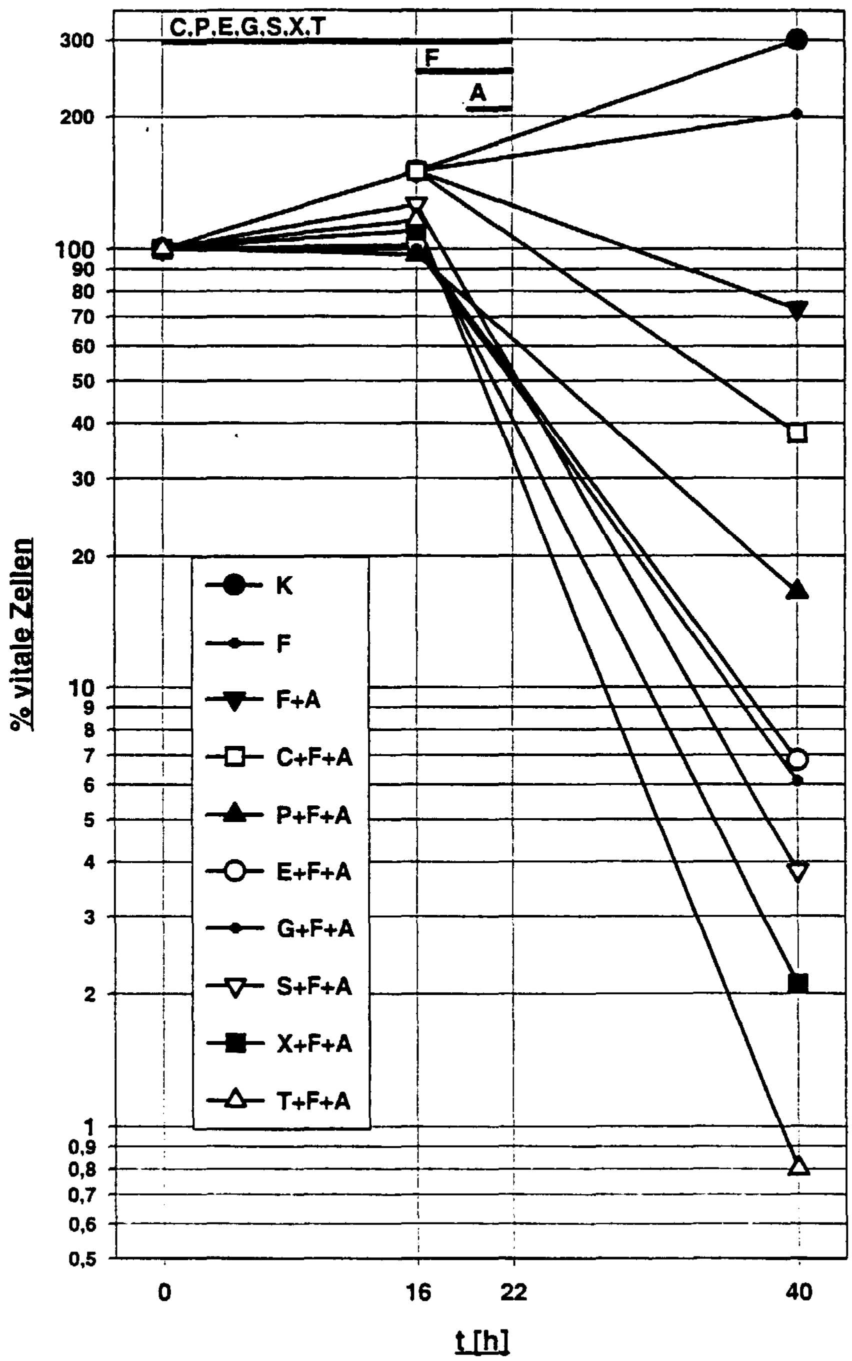

Abb. 4:
Kombinationen von 3 Substanzen
auf der Basis T+F

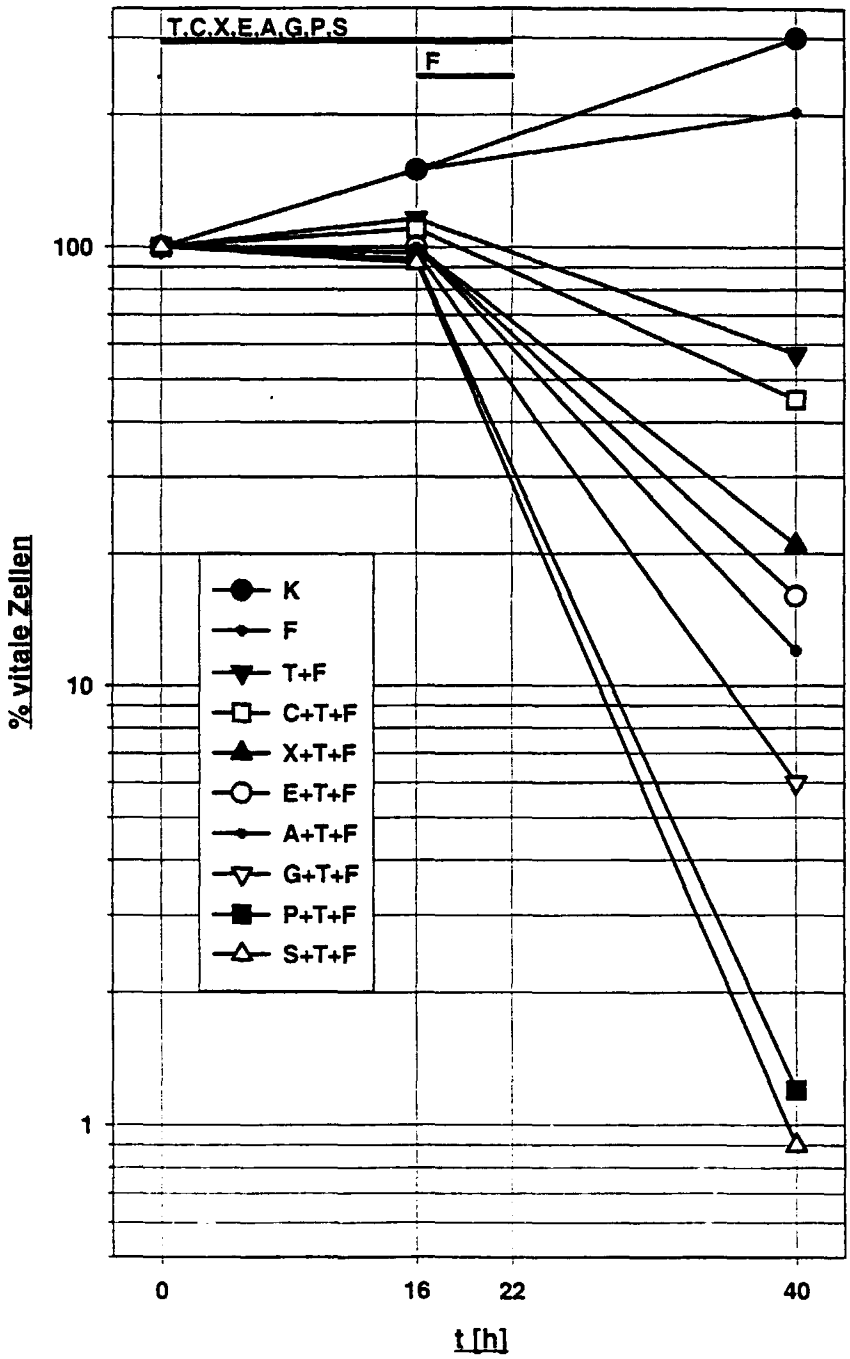

## Abb. 5: Kombinationen von 4 bzw. 5 Substanzen auf der Basis G+F+A

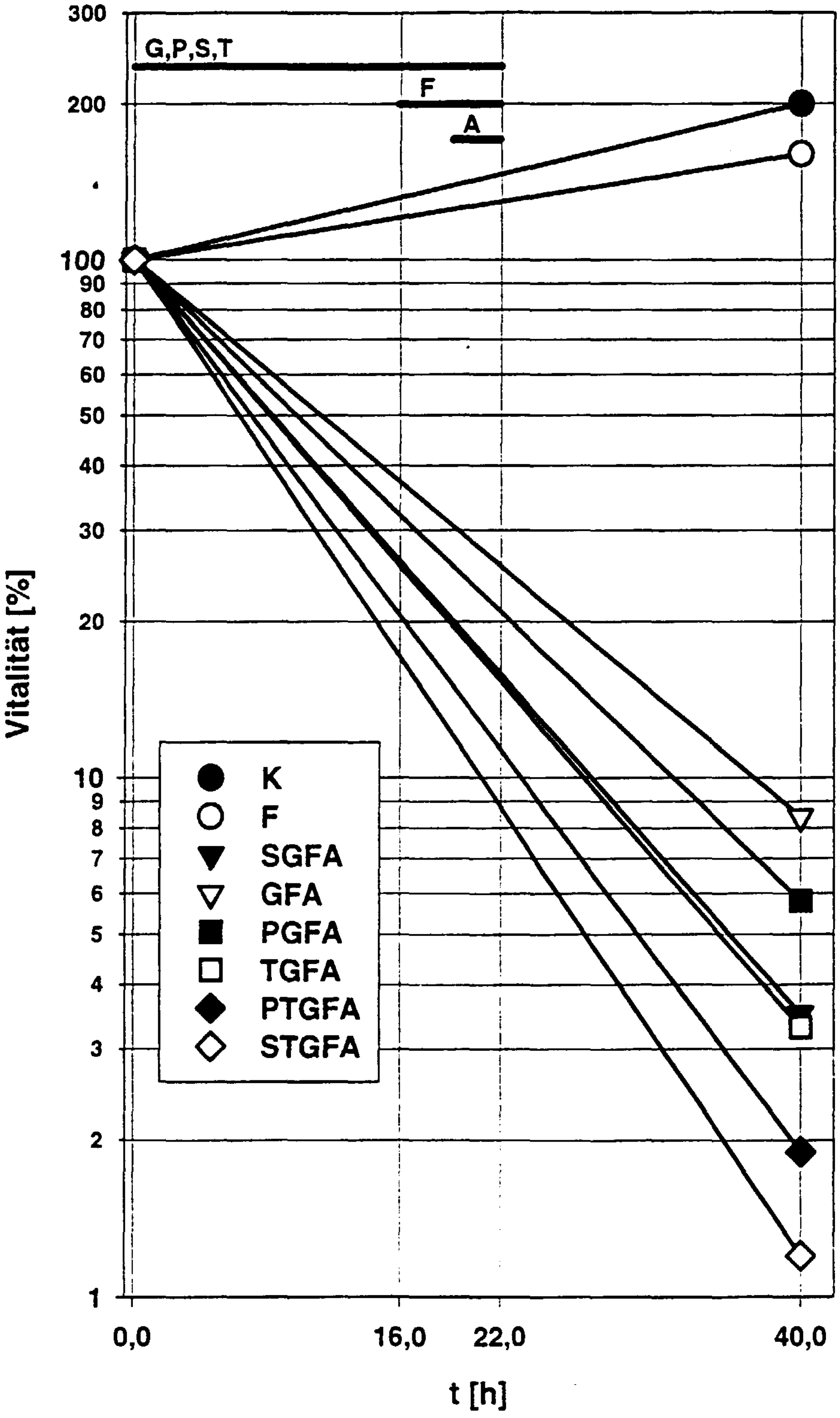

Abb. 6

Histologischer Schnitt eines unbehandelten Tumorimplantats

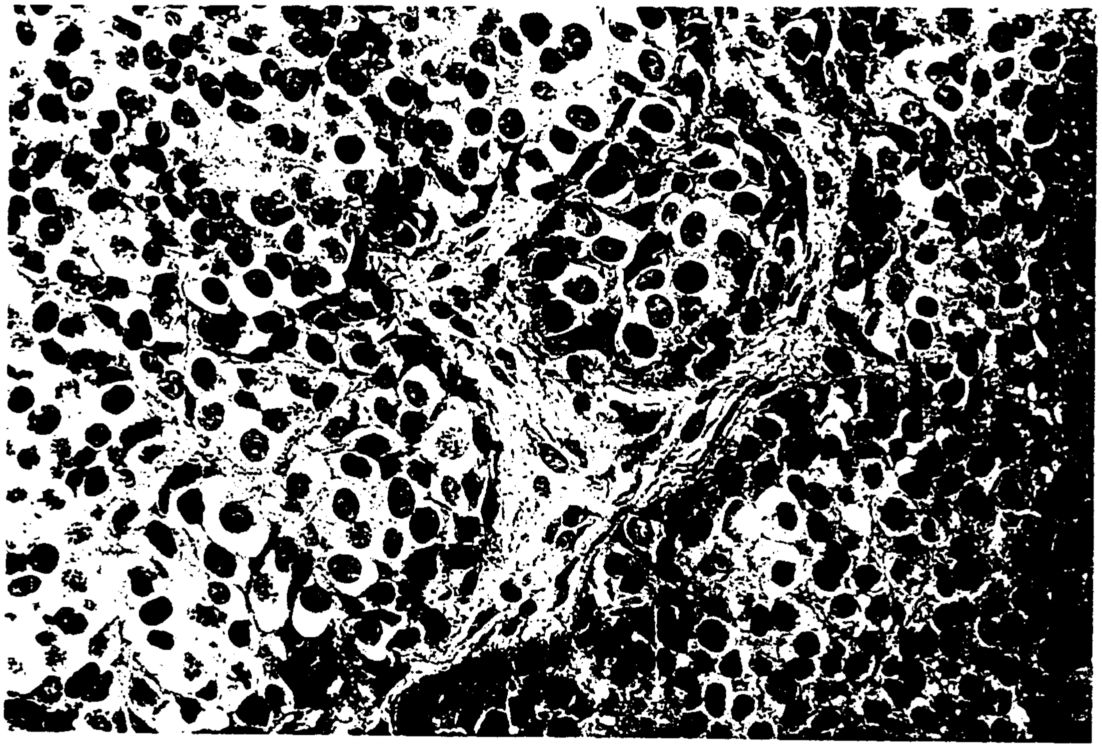

Abb. 7

Histologischer Schnitt durch ein behandeltes Tumorimplantat 24 h nach Flavopiridol-Applikation

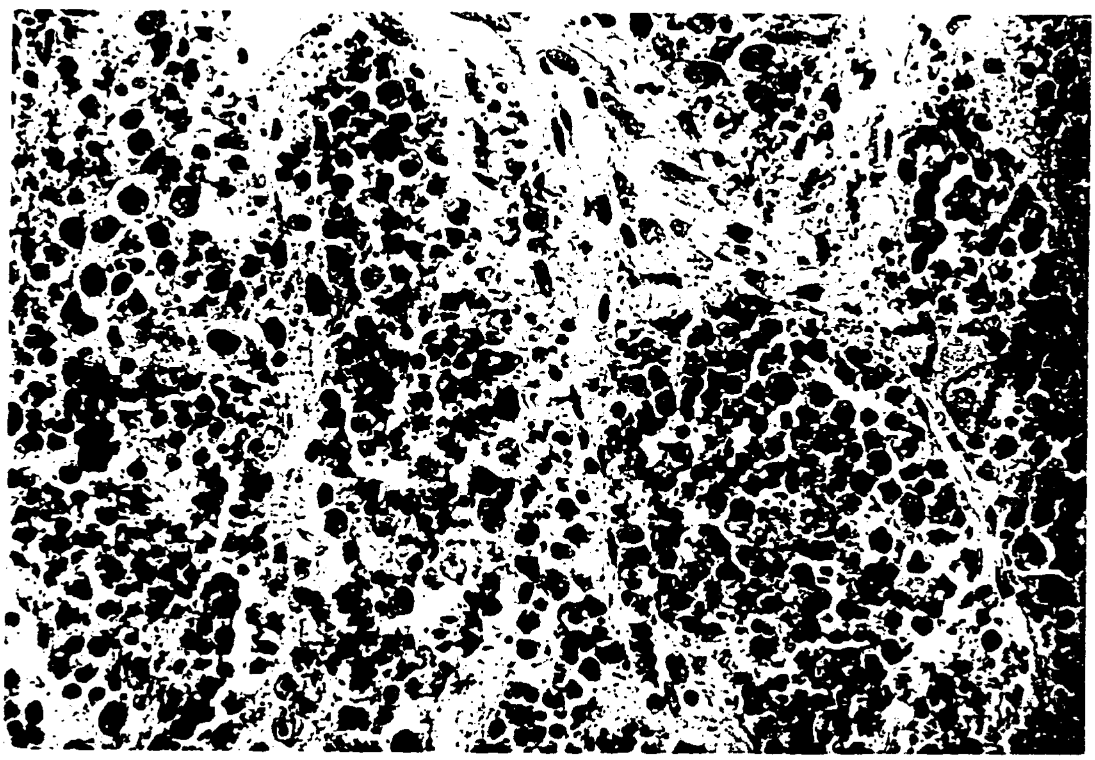

Abb. 8

Histologischer Schnitt durch ein weiteres behandeltes Tumorimplantat 24 h nach Flavopiridol-Applikation